# EUROPÄISCHE PATENTSCHRIFT

(11) **EP 3 893 813 B1**
(45) Veröffentlichungstag und Bekanntmachung des Hinweises auf die Patenterteilung: **31.01.2024**
(21) Anmeldenummer: 19818058.0
(22) Anmeldetag: 10.12.2019
(51) Int. Cl.: A61F 2/60, A61F 2/68, A61F 2/50, A61F 2/76

(54) **DÄMPFERSYSTEM**
DAMPER SYSTEM
SYSTÈME D'AMORTISSEMENT

(30) Priorität: 11.12.2018 DE 102018131698
(43) Veröffentlichungstag der Anmeldung: 20.10.2021
(73) Patentinhaber: Ottobock SE & Co. KGaA, 37115 Duderstadt (DE)
(72) Erfinder: GROHS, Thorsten, 37120 Bovenden (DE); SCHUH, Andreas, 37115 Duderstadt (DE); BREMER, Michael, 37083 Göttingen (DE); KRUSE, Andreas, 37339 Worbis (DE)
(74) Vertreter: Gramm, Lins & Partner Patent- und Rechtsanwälte PartGmbB
(86) Internationale Anmeldenummer: PCT/EP2019/084514
(87) Internationale Veröffentlichungsnummer: WO 2020/120509

(56) Entgegenhaltungen:
- EP-A2- 0 309 441
- EP-B1- 0 309 441
- DE-A1-102009 056 074
- US-B1- 6 706 074
- US-B2- 8 731 716

## Beschreibung

Die Erfindung betrifft ein Dämpfersystem für orthopädietechnische Einrichtungen mit einer proximalen Befestigungseinrichtung und einer distalen Befestigungseinrichtung, die zueinander verlagerbar miteinander gekoppelt sind, mit einer ersten, zwischen den Befestigungseinrichtungen angeordneten hydraulischen oder pneumatischen Dämpfereinrichtung mit einer Extensionskammer und einer Flexionskammer, die voneinander durch einen beweglichen Kolben getrennt und durch zumindest eine Überströmleitung strömungstechnisch miteinander verbunden sind.

Pneumatische oder hydraulische Dämpfereinrichtungen für orthopädietechnische Einrichtungen werden eingesetzt, um Relativbewegungen zwischen Komponenten der orthopädietechnischen Einrichtungen zu beeinflussen. Orthopädietechnische Einrichtungen sind beispielsweise Orthesen, Exoskelette oder Prothesen, die an einem Patienten befestigt werden. Die orthopädietechnischen Einrichtungen weisen relativ zueinander verlagerbare, insbesondere verschwenkbare oder verschiebbare Komponenten auf, deren Relativbewegung durch ein Dämpferelement beeinflusst werden kann. Beispielsweise werden Verschwenkbewegungen durch Gelenke zwischen einem Oberteil und einem Unterteil ermöglicht. Diese Verschwenkbewegungen, die zur Extension und Flexion des Gelenkes dienen, werden durch Hydraulikdämpfer oder Fluiddämpfer beeinflusst. Ebenfalls werden Verschiebebewegungen zwischen zwei Komponenten durch eine entsprechend angeordnete Dämpfereinrichtung gedämpft. Eine hydraulische Dämpfung oder eine pneumatische Dämpfung erfolgt in der Regel durch einen beeinflussten Transport von Fluid aus einer Flexionskammer oder Extensionskammer. Bei geschlossenen Systemen wird das Fluid aus der Extensionskammer in die Flexionskammer und zurück transportiert. Bei pneumatischen Systemen kann die Dämpfung über eine Kompression und/oder einen kontrollierten Auslass aus der jeweiligen Kammer erfolgen.

Aus der DE 10 2007 032 090 A1 ist ein orthopädietechnischer Fluiddämpfer bekannt, der zur Verwendung in einer Orthese oder Prothese ausgebildet ist. In einem Zylindergehäuse ist eine Verdrängungskammer ausgebildet, in dem ein Kolben gelagert ist. Ein Fluidreservoir für ein Fluid ist über eine Rückstromleitung mit der Verdrängungskammer verbunden. Ein Ventil, das eine Öffnungsstellung und eine Schließstellung, in der die Rückströmleitung zumindest teilweise verschlossen ist, einnehmen kann, ist in der Rückströmleitung angeordnet.

Aus der EP 654254 A1 ist ein Prothesengelenk mit einem Gelenkoberteil und einem Gelenkunterteil bekannt, die verschwenkbar miteinander verbunden sind. Eine Dämpfereinrichtung ist in eines der beiden Gelenkteile integriert und als Rotationshydraulik ausgebildet. Ein Drehkolben unterteilt eine Verdrängerkammer in zwei Teilkammern, die über zwei parallel geschaltete, entgegengesetzt wirkende Drosselrückschlagventile miteinander verbunden sind, wobei Drosselstellen separat von außen ansteuerbar sind, um die Dämpfung einzustellen oder zu verändern.

Die EP 672 398 B1 betrifft eine Schwenkverbindung zwischen Teilen eines orthopädischen Hilfsmittels mit einer mehrgliedrigen, kinematischen Gelenkkette mit mindestens vier Gelenkgliedern, die untereinander jeweils über eine gemeinsame Drehachse verbunden sind. Die Drehachsen sind im Wesentlichen parallel zueinander orientiert. Ein beugeseitig angeordnetes Gelenkglied ist mit seinem unteren Ende über eine zwischengeschaltete Gelenkverbindung mit einem unteren Anschlussgelenkglied drehbar verbunden, wobei die zwischengeschaltete Gelenkverbindung zwei Drehverbindungen an dem beugeseitigen Gelenkglied und zwei Drehverbindungen an dem unteren Anschlussgelenkglied aufweist, so dass bei einer Bewegung der Schwenkverbindung das beugeseitig angeordnete Gelenkglied gegenüber dem unteren Anschlussgelenkglied eine zwangsläufige Bewegung ausführt, die sowohl eine Translation als auch eine Rotation umfasst. Zumindest ein federndes Element kann zwischen zwei Gelenkgliedern angeordnet sein, das federnde Element kann einen Dämpfer enthalten.

Die EP 3 089 722 B1 betrifft ein Prothesenkniegelenk mit einem Oberteil, einem Unterteil und einem viergliedrigen Gelenksystem, das an dem Oberteil angeordnet ist. Das Gelenksystem ist entgegen einer Federkraft während einer Standphasenflexion verschwenkbar an dem Unterteil gelagert. Ein erstes Federelement federt die Bewegung des Gelenksystems gegenüber dem Unterteil um eine Schwenkachse ab. Ein zweites Federelement beeinflusst die Bewegung innerhalb des Gelenksystems. Die Federelemente können jeweils als Feder-Dämpfer-Element oder Feder-Dämpfer-System ausgebildet sein, wobei die Dämpfung über einen Fluiddämpfer oder einen Elastomerdämpfer bereitgestellt werden kann. Das Elastomerelement kann als Tubus ausgebildet sein oder zumindest einen Tubus aufweisen.

Die EP 0 309 441 A2 zeigt einen doppelt wirkenden hydraulischen Dämpfer für ein künstliches Gelenk. Ein Hydraulikzylinder ist dabei von einem Gummibalg umgeben, der an der Kolbenstange und einer Basis befestigt ist. Der Balg dient zur Aufnahme der Hydraulikflüssigkeit aus dem Zylinder.

Die US 8 731 716 B2 betrifft ein Verfahren zum Betreiben eines biomimetischen mechanischen Gelenks, das eine Mehrzahl von, beispielsweise hydraulischen Aktuatoren aufweist. Die Hysterese beim Umschalten von einem Aktuator zum anderen ist einstellbar.

Die US 6 706 074 B1 beschreibt eine künstliche Kniegelenkeinrichtung mit einer Mehrzahl von Dämpfungselementen. In einem Hydraulikzylinder ist eine Schraubenfeder als zusätzliches Federelement angeordnet. Dem Hydraulikzylinder ist ein Ölspeicherbehälter zugeordnet, der bspw. neben dem Hydraulikzylinder angeordnet ist.

Die DE 10 2009 056 074 A1 beschreibt eine Knieexartikulationsprothese mit drei Hydraulikzylindern, von denen zwei zueinander parallel geschaltet sind.

Aufgabe der vorliegenden Erfindung ist es, ein Dämpfersystem für orthopädietechnische Einrichtungen bereitzustellen, insbesondere für Gelenkeinrichtungen der unteren Extremität, mit dem es möglich ist, das Dämpfungsverhalten besser auf den jeweiligen Einsatzzweck abzustimmen und insbesondere eine hohe Ausfallsicherheit zu erreichen.

Erfindungsgemäß wird diese Aufgabe durch ein Dämpfersystem mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das erfindungsgemäße Dämpfersystem für orthopädietechnische Einrichtungen mit einer proximalen Befestigungseinrichtung und einer distalen Befestigungseinrichtung, die zueinander verlagerbar miteinander gekoppelt sind, mit einer ersten, zwischen den Befestigungseinrichtungen angeordneten hydraulischen oder pneumatischen Dämpfereinrichtung mit einer Extensionskammer und einer Flexionskammer, die voneinander durch einen beweglichen Kolben getrennt und durch zumindest eine Überströmleitung strömungstechnisch miteinander verbunden sind, sieht vor, dass zwischen den Befestigungseinrichtungen eine parallel zu der ersten Dämpfereinrichtung wirkende zweite Dämpfereinrichtung mit einem geschwindigkeitsunabhängigen Hystereseverhalten angeordnet ist. Während hydraulische oder pneumatische Dämpfereinrichtungen ein geschwindigkeitsabhängiges Hystereseverhalten aufweisen, wird es durch eine Parallelschaltung oder eine parallel wirkende Anordnung einer zweiten Dämpfereinrichtung ermöglicht, unterschiedliche Dämpferverhalten miteinander zu kombinieren und für unterschiedliche Bewegungsphasen oder Belastungsphasen unterschiedliche Dämpfungseigenschaften bereitzustellen. So ist es beispielsweise möglich, bei Orthesen-Kniegelenken oder Prothesen-Kniegelenken in der Standphase ein Einsinken oder eine Flexion zu ermöglichen. Bei einer gleichzeitigen Dämpfungswirkung der beiden Dämpfereinrichtungen können sich geschwindigkeitsabhängig unterschiedliche Dämpferverhalten in Extensionsrichtung und in Flexionsrichtung ergeben, so dass sich in Abhängigkeit von der Bewegungsausführung unterschiedliche Dämpferverhalten erreichen und einstellen lassen. Darüber hinaus ist bei einem Versagen der hydraulischen oder pneumatischen Dämpfereinrichtung stets die weitere Dämpfereinheit vorhanden, so dass ein Notbetrieb mit ausreichenden, gegebenenfalls jedoch nicht mehr optimalen Dämpfereigenschaften gewährleistet bleiben kann. Die Hydraulikdämpfung oder Pneumatikdämpfung ist über einen weiten Dämpfungsbereich einstellbar und kann einfach durch Verringerung oder Vergrößerung des Strömungsquerschnittes an dem jeweiligen Patienten oder Einsatzzweck angepasst werden. Die insofern leistungsfähigere und variablere Hydraulik- oder Pneumatikdämpfung hat aufgrund der beweglichen Komponenten eine höhere Ausfallwahrscheinlichkeit, die durch die parallel wirkende zweite Dämpfereinrichtung aufgefangen werden kann.

Die zweite Dämpfereinrichtung kann als geschwindigkeitsunabhängiger Dämpfer aufgebaut sein, insbesondere als Reibungsdämpfungsdämpfer. Jedoch kann auch eine Ausgestaltung des Dämpfers als ein Strukturdämpfer oder Elastomerdämpfer ein Hystereseverhalten aufweisen, das im Wesentlichen unabhängig von der Verformungsgeschwindigkeit der Dämpfereinrichtung ist. Wichtig ist, dass die Energieaufnahme im Wesentlichen unabhängig von der Verformungsgeschwindigkeit des Elastomers ist, so dass mit der zweiten Dämpfereinrichtung eine weitere, andere Dämpfercharakteristik zur Verfügung gestellt werden kann. Die Verformungswege und damit die Energieaufnahme bei konstanter Federrate sind dabei unabhängig von der Verformungsgeschwindigkeit.

In einer Weiterbildung der Erfindung ist die zweite Dämpfereinrichtung als Strukturdämpfer aus einem Elastomer ausgebildet, wodurch neben der Dämpferwirkung der zweiten Dämpfereinrichtung auch eine Kraftspeicherung und eine Rückstellkraft bereitgestellt werden. Die zweite Dämpfereinrichtung ist als kombiniertes Feder-Dämpfer-Element ausgebildet, so dass nicht nur die Relativbewegung zwischen Oberteil und Unterteil oder den beiden Befestigungseinrichtungen abgebremst wird, sondern auch eine Rückstellkraft gegen ein Einsinken oder eine weitere Verlagerung der Komponenten, zwischen denen das Dämpfersystem angeordnet ist, bereitgestellt werden kann. Die zweite Dämpfereinrichtung als Strukturdämpfer kann aus einem Kunststoffkomposit ausgebildet sein, so dass die zweite Dämpfereinrichtung als elastischer Festkörper ausgebildet ist, der ein gleichbleibendes und geschwindigkeitsunabhängiges Rückstellverhalten aufweist.

Eine Weiterbildung der Erfindung sieht vor, dass die zweite Dämpfereinrichtung die erste Dämpfereinrichtung umfänglich umgibt, wodurch bei einer Undichtigkeit insbesondere eines Hydraulikdämpfers vermieden wird, dass Hydraulikfluid in die Umgebung austritt oder erreicht wird, dass austretendes Hydraulikfluid aufgefangen oder zumindest aufgehalten wird. Die zweite Dämpfereinrichtung kann die erste Dämpfereinrichtung vollumfänglich umgeben und einschließen. Die erste Dämpfereinrichtung kann in der zweiten Dämpfereinrichtung aufgenommen und dichtend oder abgedichtet daran festgelegt sein, so dass sich ein Modul ergibt, das eine hohe Dauersicherheit und Verfügbarkeit gewährleistet.

Die erste Dämpfereinrichtung kann in ihrem Dämpfungsverhalten einstellbar ausgebildet sein. Die Einstellung kann beispielsweise einmalig erfolgen, durch Einstellen von Ventilen oder Drosseln oder aber während der Benutzung des Dämpfersystems über Aktuatoren, wie Motoren oder andere Stelleinrichtungen, die beispielsweise sensorgesteuert oder mechanische in Abhängigkeit von Bewegungen oder Verlagerungssituationen der Befestigungseinrichtungen aufeinander zu oder voneinander weg erfolgen können. Beispielsweise kann in Abhängigkeit von einem gemessenen Gelenkwinkel oder einer Bewegungsrichtung ein Ventil oder eine Drossel geöffnet oder geschlossen oder der jeweilige Strömungsquerschnitt vergrößert oder verringert werden.

Die zweite Dämpfereinrichtung kann als Tubusdämpfer ausgebildet sein oder zumindest einen Tubus aufweisen, wobei es möglich ist, mehrere Tubuselemente miteinander zu kombinieren und in Reihe anzuordnen.

In einer Weiterbildung der Erfindung ist vorgesehen, dass der Tubusdämpfer zumindest einen nach außen gewölbten, tonnenförmigen oder glockenförmigen Abschnitt aufweist, über dessen Formgebung und Auslegung, beispielsweise in der Höhe, der Wölbung oder in der Materialdicke, die Dämpfung, das Federungsverhalten sowie das Hystereseverhalten einstellbar sind. Durch eine geeignete Auswahl des zweiten Dämpferelementes als entsprechend ausgebildeter und geformter Tubusdämpfer mit den jeweiligen tonnenförmigen oder glockenförmigen Abschnitten kann in Zusammenspiel mit der ersten Dämpfereinrichtung eine Anpassung an den jeweiligen Einsatzzweck in einem großen Umfang erfolgen. Der Tubusdämpfer kann mehrere nach außen gewölbte, tonnenförmige und/oder glockenförmige Abschnitte mit unterschiedlichen Durchmessern, Materialstärken und/oder unterschiedlichen Höhen aufweisen, so dass jeder Tubusabschnitt eine unterschiedliche Dämpfer- und/oder Feder-Charakteristik aufweist, wodurch sich eine hohe Variationsbreite an Feder- und Dämpferverhalten erreichen lässt.

Der Tubusdämpfer kann einen nach innen und/oder außen abstehenden Abstützabschnitt aufweisen, der an einem Gehäuse, insbesondere an einer Außenseite und/oder Innenseite eines Gehäuses der ersten Dämpfereinrichtung anliegt. Über den nach innen und/oder außen abstehenden Abstützabschnitt ist es möglich, eine Führung des Tubusdämpfers an der Außenseite und/oder Innenwand eines Gehäuses der ersten Dämpfereinrichtung zu ermöglichen. Darüber hinaus wird die erste Dämpfereinrichtung durch einen sie außen umgebenen Tubusdämpfer geschützt und eine kompakte Bauweise ermöglicht.

In einer Weiterbildung der Erfindung ist der ersten Dämpfereinrichtung ein Schaltelement zugeordnet, das den Strömungswiderstand in der Extensionsrichtung oder Flexionsrichtung zumindest reduziert und in der entgegengesetzten Richtung auf einem eingestellten Strömungswiderstand hält. Dieses Schaltelement kann beispielsweise dazu eingesetzt werden, eine richtungsabhängige Zuschaltung bzw. Abschaltung der ersten Dämpfereinrichtung zu ermöglichen. Es wird beispielsweise für die Flexion einer Gelenkeinrichtung ein geringerer Widerstand oder eine geringere Dämpfung benötigt, wird die erste Dämpfereinrichtung ausgeschaltet oder in ihrer Wirkung reduziert, so dass beispielsweise die Pneumatik oder Hydraulik überbrückt wird. In Flexionsrichtung wirkt dann nur noch die zweite Dämpfereinrichtung oder der Strukturdämpfer aus einem Elastomer, während in der umgekehrten Bewegung sowohl die zweite Dämpfereinrichtung als auch die erste Dämpfereinrichtung in Gestalt der hydraulischen oder pneumatischen Dämpfereinrichtung aktiviert wird. Gleiches kann auch umgekehrt erfolgen, also eine doppelte Dämpferwirkung im Flexionsrichtung und eine einfache Dämpferwirkung in Extensionsrichtung. Diese Umschaltung kann bewegungsrichtungsabhängig und/oder belastungsabhängig erfolgen. Die Schalteinrichtung kann beispielsweise als ein Umschaltventil ausgebildet sein, das strömungsrichtungsabhängig geschaltet wird.

In Kompressionsrichtung der zweiten Dämpfereinrichtung kann der Strömungswiderstand der ersten Dämpfereinrichtung bevorzugt reduziert werden.

Eine Weiterbildung der Erfindung sieht vor, dass das Hystereseverhalten der ersten Dämpfereinrichtung härter als das Hystereseverhalten der zweiten Dämpfereinrichtung ist. Die zweite Dämpfereinrichtung weist dabei eine weichere Hysteresekurve auf, also eine größere Abweichung zwischen dem Dämpfungsverhalten bei der Einfederung im Vergleich zum Dämpfungsverhalten der Ausfederung.

In der Befestigungseinrichtung kann ein Zylinder zur Aufnahme des Kolbens der ersten Dämpfereinrichtung ausgebildet sein, um eine kompakte Bauweise des Dämpfersystems zu ermöglichen.

Eine Variante der Erfindung sieht vor, dass die zweite Dämpfereinrichtung innerhalb des Mediums der ersten hydraulischen oder pneumatischen Dämpfereinrichtung angeordnet ist, also innerhalb des Fluids, das zwischen der Extensionskammer und der Flexionskammer bei einer Verlagerung der Befestigungseinrichtungen zueinander bewegt wird.

Eine Weiterbildung der Erfindung sieht vor, dass mindestens einer der nach außen gewölbten, tonnenförmigen und/oder glockenförmigen Abschnitte, die auch unterschiedliche Durchmesser und/oder unterschiedliche Höhen aufweisen können, oberhalb der ersten Dämpfereinrichtung angeordnet ist, während mindestens einer der nach außen gewölbten, tonnenförmigen und/oder glockenförmigen Abschnitte unterhalb der ersten Dämpfereinrichtung angeordnet ist. Der Tubusstrukturdämpfer ist somit zweigeteilt, ein Teil ist oberhalb und ein Teil ist unterhalb der ersten Dämpfereinrichtung angeordnet.

Nachfolgend werden Ausführungsbeispiele der Erfindung anhand der beigefügten Figuren näher erläutert. Es zeigen:
- Figur 1 -: eine Außenansicht eines Ausführungsbeispiels; sowie
- Figur 2 -: eine Schnittansicht durch ein Dämpfersystem gemäß Figur 1;
- Figur 3 -: ein Dämpfersystem im eingebauten Zustand;
- Figur 4 -: ein Dämpfersystem in einem Prothesenkniegelenk mit Standphasenflexion;
- Figur 5 -: ein Prothesenkniegelenk gemäß Figur 4 im flektierten Zustand;
- Figur 6 -: das Prothesenkniegelenk gemäß Figur 4 in extendierter Stellung;
- Figur 7 -: das Prothesenkniegelenk gemäß Figur 6 mit freigeschnittenem Dämpfersystem;
- Figur 8 -: Figur 7 mit Dämpfersystem in Schnittansicht;
- Figur 9 -: eine Variante der Erfindung mit einem Tubusstrukturdämpfer in der ersten Dämpfereinrichtung; sowie
- Figur 10 -: eine Variante der Figur 9.

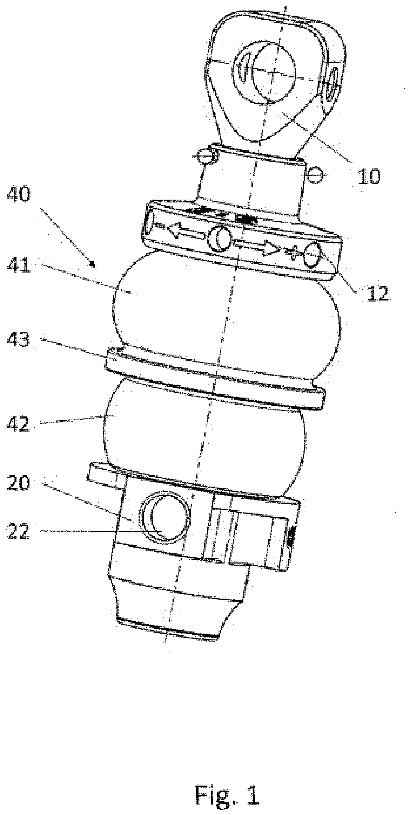

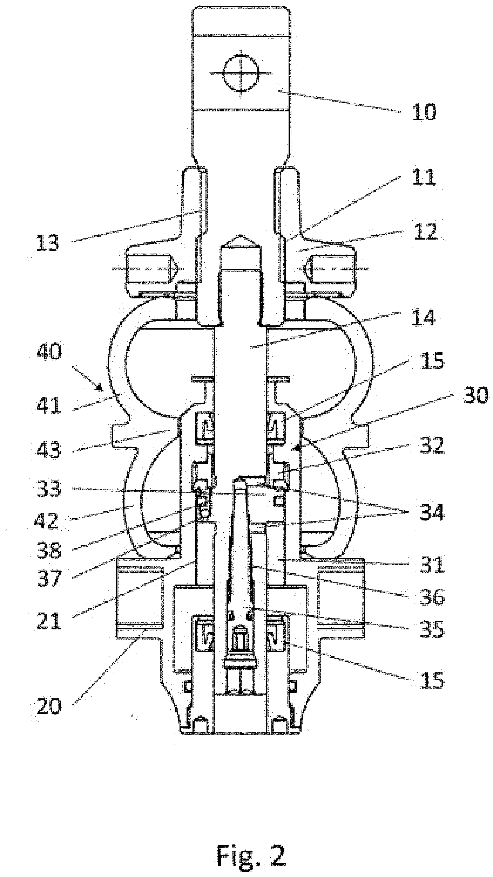

Figur 1 zeigt in einer perspektivischen Darstellung ein Dämpfersystem für orthopädietechnische Einrichtungen, beispielsweise für Prothesen, Orthesen oder Exoskelette, um Relativbewegungen zwischen zwei Komponenten der orthopädietechnischen Einrichtung zu dämpfen und um gegebenenfalls Rückstellbewegungen einzuleiten. Das Dämpfersystem weist eine proximale Befestigungseinrichtung 10 auf, über die das Dämpfersystem an einer Komponente der orthopädietechnischen Einrichtung festgelegt werden kann. Die proximale Befestigungseinrichtung 10 weist eine Durchgangsbohrung auf, durch die ein Bolzen oder eine Schraube hindurchgeführt werden kann, um eine reversible Befestigung bei gleichzeitiger Verschwenkbarkeit um die Bolzenachse herum zu ermöglichen. An der proximalen Befestigungseinrichtung 10 ist ein Außengewinde angeordnet oder ausgebildet, in das ein Innengewinde an einem Vorspannelement 12 eingreift. Das Vorspannelement 12 ist drehbar an der proximalen Befestigungseinrichtung 10 gelagert, so dass durch Drehen in die eine oder andere Richtung eine Verlagerung des Vorspannelementes entlang der Längserstreckung des Dämpfersystems ausgeführt wird. Das Vorspannelement 12 liegt an einem oberen Ende einer zweiten Dämpfereinrichtung 40 an, die als Strukturdämpfer aus einem Elastomer ausgebildet ist. Die zweite Dämpfereinrichtung 40 ist als Tubusdämpfer mit zwei glockenförmigen oder tonnenförmigen Abschnitten 41, 42 ausgebildet, die in Reihe hintereinander entlang der Längserstreckung des Dämpfersystems angeordnet sind. Zwischen den beiden tonnenförmigen, nach außen gewölbten Abschnitten 41, 42 ist ein Abstützabschnitt 43 angeordnet, der in Gestalt einer Scheibe ausgebildet ist. Der weitere Aufbau der zweiten Dämpfereinrichtung 40 wird anhand der Figur 2 erläutert werden.

Das distale Ende der zweiten Dämpfereinrichtung 40 liegt auf einem Absatz einer distalen Befestigungseinrichtung 20 auf. Die distale Befestigungseinrichtung 20 weist in dem dargestellten Ausführungsbeispiel zwei Aufnahmen 22 mit jeweils einem Innengewinde zur Aufnahme von Bolzen oder Schrauben auf, um die zweite oder distale Befestigungseinrichtung 20 an einer weiteren Komponente der orthopädietechnischen Einrichtung festlegen zu können.

In der Figur 2 ist in einer Längsschnittdarstellung das Dämpfersystem gemäß Figur 1 dargestellt. Die proximale Befestigungseinrichtung 10 mit der Durchgangsbohrung zur Aufnahme eines Bolzens oder einer Schraube ist zu erkennen. In dem Bolzenauge kann eine weitere, senkrecht zu der ersten Durchgangsbohrung orientierte Bohrung angeordnet sein, beispielsweise um die proximale Befestigungseinrichtung gegen eine Verdrehung um die Bolzenachse zu sichern.

Von der Durchgangsbohrung erstreckt sich in Längserstreckung des Dämpfersystems ein Bolzen, auf dessen Außenseite das Außengewinde 11 ausgebildet ist, das in Eingriff mit einem Innengewinde 13 des Vorspannelementes 12 steht. Das Vorspannelement 12 liegt an dem proximalen, oberen Ende der zweiten Dämpfereinrichtung 40 in Gestalt eines Tubusdämpfers an. Innerhalb des Bolzens der proximalen Befestigungseinrichtung 10 ist eine Kolbenstange 14 eingeschraubt. Die Kolbenstange 14 ist beweglich in einem Zylinder 21 geführt, der in der distalen Befestigungseinrichtung 20 ausgebildet ist. An der Kolbenstange 14 ist ein Kolben 33 angeordnet oder ausgebildet, der sich in dem Zylinder 21 entlang der Längserstreckung des Dämpfersystems oder der Kolbenstange 14 reziprok bewegen lässt. Dadurch ist es möglich, die proximale Befestigungseinrichtung 10 in Richtung auf die distale Befestigungseinrichtung 20 und zurück zu bewegen.

Die distale Befestigungseinrichtung 20 bildet ein Gehäuse aus, in dem die Kolbenstange 14 geführt und das an seinem proximalen Ende über eine Dichtung 15 abgedichtet ist. Die Dichtung 15 liegt an der Kolbenstange 14 an und verhindert ein Austreten eines Hydraulikfluids, das innerhalb des Zylinders 21 in einer Extensionskammer 32 und einer Flexionskammer 31 angeordnet ist. Die Extensionskammer 32 wird von der Flexionskammer 31 durch den Kolben 33 getrennt. Innerhalb der Kolbenstange 14 ist eine Überströmleitung 34 ausgebildet, durch die bei einer Verlagerung des Kolbens 33 aufgrund einer Bewegung der Kolbenstange 14 Hydraulikfluid von der Extensionskammer 32 in die Flexionskammer 31 strömen kann, wenn die beiden Befestigungseinrichtungen 10, 20 aufeinander zu bewegt werden. Wird die Bewegung umgekehrt, also die Befestigungseinrichtungen 10, 20 voneinander weg bewegt, strömt Hydraulikfluid von der Flexionskammer 31 durch die Überströmleitung 34 in die Extensionskammer 32. Innerhalb der Überströmleitung 34, die in dem dargestellten Ausführungsbeispiel durch die Kolbenstange 14 und den Kolben 33 hindurchführt, ist eine Düsennadel 35 angeordnet, die an der Außenseite über ein Gewinde 36 in der Kolbenstange 34 einstellbar gelagert ist. Durch Verdrehen der Düsennadel 35 ist es möglich, den Strömungsquerschnitt der Überströmleitung 34 zu verändern. Dazu ist an der distalen Stirnseite der distalen Befestigungseinrichtung 20 eine Zugangsöffnung ausgebildet, durch die über ein Werkzeug, beispielsweise einen Schraubendreher, einen Vielzahnschlüssel oder einen Innensechskantschlüssel eine Verdrehung der Düsennadel 35 erfolgen kann. In dem distalen Endbereich der distalen Befestigungseinrichtung 20 ist eine weitere Dichtung 15 zur Abdichtung der Kolbenstange 14 nach außen angeordnet.

Die Kolbenstange 14 bildet zusammen mit dem Kolben 33, dem Zylinder 21 und der Überströmöffnung 34 eine erste, weitere Dämpfereinrichtung 30 aus, die als hydraulische Dämpfereinrichtung 30 ausgebildet ist. Die erste, hydraulische Dämpfereinrichtung 30 wirkt parallel zu der zweiten Dämpfereinrichtung 40, die zwischen dem Vorspannelement 12 und dem Absatz an der distalen Befestigungseinrichtung 20 angeordnet ist.

Die zweite Dämpfereinrichtung 40 ist als Tubusdämpfer aus einem Elastomermaterial ausgebildet und weist zwei tonnenförmigen, hohle Abschnitte 41, 42 auf, die in Reihe zueinander geschaltet sind. Der proximale Tubusabschnitt 41 weist im Vergleich zu dem distalen Tubusabschnitt 42 einen größeren Außendurchmesser und einen größeren Innendurchmesser sowie eine geringere Längserstreckung oder Höhe auf. Die Wandstärke der beiden Abschnitte 41, 42 ist im Wesentlichen gleich und gleichbleibend, in den proximalen und distalen Endbereichen der Abschnitte 41, 42 vergrößern sich die Wandstärken. Zwischen den beiden Abschnitten 41, 42 ist ein massiver Abstützabschnitt 43 ausgebildet, der einen Außendurchmesser aufweist, der im Wesentlichen dem maximalen Außendurchmesser des proximalen Abschnittes 41 entspricht. Der Abstützabschnitt 43 erstreckt sich bis zur Außenwand des Zylindergehäuses, das durch das distale Befestigungselement 20 ausgebildet wird. Der Abstützabschnitt 43 bietet eine radiale Führung für den Tubusdämpfer oder das zweite Dämpferelement 40, wenn die beiden Befestigungseinrichtungen 10, 20 aufeinander zu bewegt werden. Bei einer Verlagerung der Befestigungseinrichtungen 10, 20 aufeinander zu werden neben einer Bewegung des Kolbens 33 innerhalb des Zylinders 21 die beiden tonnenförmigen Abschnitte 41, 42 komprimiert. Aufgrund der Hohlkörperstruktur der beiden Abschnitte 41, 42 wölben diese sich nach außen, der Abstützabschnitt 43 bewegt sich entlang einer Wand an der Außenseite des Zylinders 21 oder entlang einer Wand des Gehäuses für die hydraulische erste Dämpfereinrichtung 30 nach unten in Richtung auf die distale Befestigungseinrichtung 20. Somit tritt neben einer hydraulischen Dämpfung aufgrund der behinderten Fluidströmung aus der Extensionskammer 32 in die Flexionskammer 31 eine zweite, zusätzliche Dämpfung über den als Festkörper ausgebildeten Tubusdämpfer 40 bereitgestellt. Aufgrund der elastischen Ausgestaltung der zweiten Dämpfereinrichtung 30 bildet dieser ein Feder-Dämpfer-System aus, das bei Wegfall einer Kraft, die die beiden Befestigungseinrichtungen 10, 20 aufeinander zu bewegt, eine Rückstellbewegung einleitet.

Die erste Dämpfereinrichtung 30 umgibt die erste Dämpfereinrichtung 30 über den gesamten Umfang und weist ein anderes Dämpferverhalten als die zweite Dämpfereinrichtung 40 auf. Insbesondere ist die erste Dämpfereinrichtung 30 in dem Hystereseverhalten geschwindigkeitsabhängig, während die zweite Dämpfereinrichtung 40 ein geschwindigkeitsunabhängiges Hystereseverhalten aufweist. Darüber hinaus kann die erste Dämpfereinrichtung 30 schaltbar ausgestaltet sein und ist über einen weiten Bereich einstellbar. Durch Hineinschrauben oder Hinausschrauben des Nadelventils 35 ist es möglich, den Strömungsquerschnitt der Überströmleitung 34 über einen großen Bereich zu variieren, wodurch die Dämpfereigenschaften des Hydraulikdämpfers 30 entsprechend verändert werden können. Darüber hinaus kann durch ein einfaches Schaltelement 37 in Gestalt eines Rückschlagventils erreicht werden, dass in Abhängigkeit von der Bewegungsrichtung die hydraulische Dämpfereinrichtung 30 aktiv geschaltet oder umgangen wird. Bei einer Flexionsbewegung, wenn die beiden Befestigungseinrichtungen 10, 20 aufeinander zu bewegt werden, liegt ein hoher Druck auf der der Extensionskammer 32 zugewandten Seite des Kolbens 33 an. Das Rückschlagventil 37 öffnet und gibt einen Bypasskanal 38 frei, so dass unabhängig von einer Behinderung des Strömungsquerschnittes in der Überströmleitung 34 Hydraulikfluid von der Extensionskammer 32 in die Flexionskammer 31 strömen kann. Umgekehrt wird das Schaltelement 37 in Gestalt beispielsweise einer federbelasteten Kugel bei einer Extensionsbewegung, also bei einer Umkehrung und einer Vergrößerung des Abstandes der beiden Befestigungseinrichtungen 10, 20 voneinander, die Bypassleitung 38 verschlossen, so dass Hydraulikfluid von der Flexionskammer 31 nur noch durch den Überströmkanal 34 in die Extensionskammer 32 strömen kann. Somit ist es möglich, bei unterschiedlichen Bewegungen unterschiedliche Dämpfereinrichtungen zu aktivieren. Durch eine Federbelastung des Schaltelementes 37 ist es zudem möglich, eine belastungsabhängige Freigabe der Bypassleitung 38 zu erreichen.

Durch eine umgekehrte Anordnung des Schaltelementes 37 ist es möglich, eine Flexionsbewegung nur über die zweite Dämpfereinrichtung 40 zu dämpfen oder eine Verringerung der Dämpfungswirkung der ersten, hydraulischen Dämpfereinrichtung 30 bereitzustellen, während bei der umgekehrten Bewegung beide Dämpfereinrichtungen 30, 40 in einem vollen Umfang aktiviert sind. Neben einer mechanischen Lösung, wie sie in dem Ausführungsbeispiel beschrieben ist, kann auch eine elektronische Lösung des Schaltvorganges erfolgen.

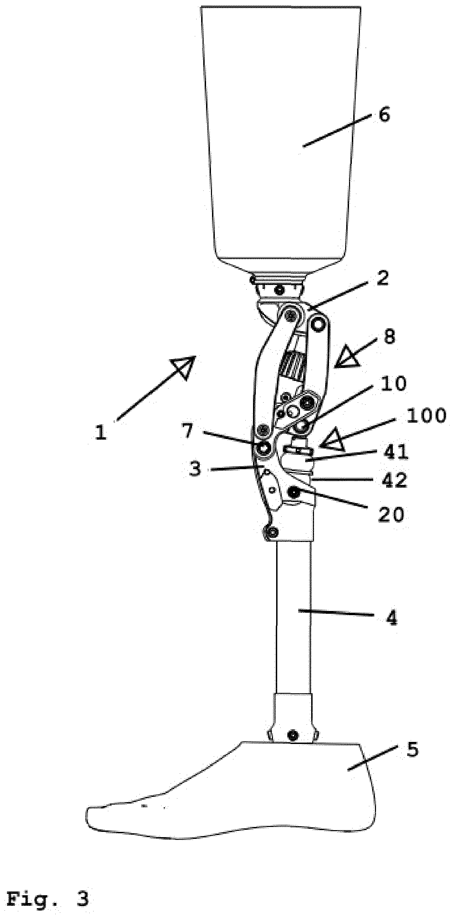

In der Figur 3 ist das Dämpfersystem 100 in einem eingebauten Zustand dargestellt. Das Dämpfersystem 100 ist Teil einer Prothese 1 in Gestalt eines Prothesenbeines mit einem Prothesenkniegelenk, das ein Oberteil 2 mit einem proximal daran angeordneten Oberschenkelschaft 6 zur Aufnahme eines Oberschenkelstumpfes aufweist. Das Oberteil 2 ist gelenkig an einem Unterteil 3 des Prothesenkniegelenkes angeordnet. Distal an dem Unterteil 3 ist ein Unterschenkelrohr 4 mit einem daran angeordneten Prothesenfuß 5 befestigt. Das Oberteil 2 ist über ein Mehrlenkersystem 8, das als Vierlenkersystem ausgebildet ist, relativ zu dem Unterteil 3 verschwenkbar. Das Mehrlenkersystem 3 ist darüber hinaus um eine separate Schwenkachse 7 an dem Unterteil 3 gelagert. Um die Schwenkachse 7, die frontal und distal zu dem Mehrlenkersystem 8 angeordnet ist, kann das gesamte Mehrlenkersystem 8 zusammen mit dem Oberteil 2 und dem Oberschenkelschaft 6 entgegen der Gehrichtung, im dargestellten Ausführungsbeispiel im Uhrzeigersinn, verschwenken. Das Mehrlenkersystem 8 und das Oberteil 2 stützen sich über das Dämpfersystem 100 gegen eine Verschwenkung um die Schwenkachse 7 ab. Dazu ist die proximale Befestigungseinrichtung 10 an dem distalen Ende des posterioren Lenkers des Mehrlenkersystems 8 befestigt. Die distale Befestigungseinrichtung 20 ist an dem Unterteil 3 befestigt, sodass bei einer Verschwenkung um die Schwenkachse 7 bei einer Standphasenflexion das gesamte Mehrlenkersystem 8 zusammen mit dem Oberteil 2 und dem Oberschenkelschaft 6 in Uhrzeigerrichtung um die Schwenkachse 7 verschwenkt, ohne dass das Mehrlenkersystem 8 mit den vier Gelenkachsen eine Relativbewegung zueinander ausführen würde. In der Figur 3 ist die Prothese 1 in einem maximal extendierten, unbelasteten Zustand dargestellt.

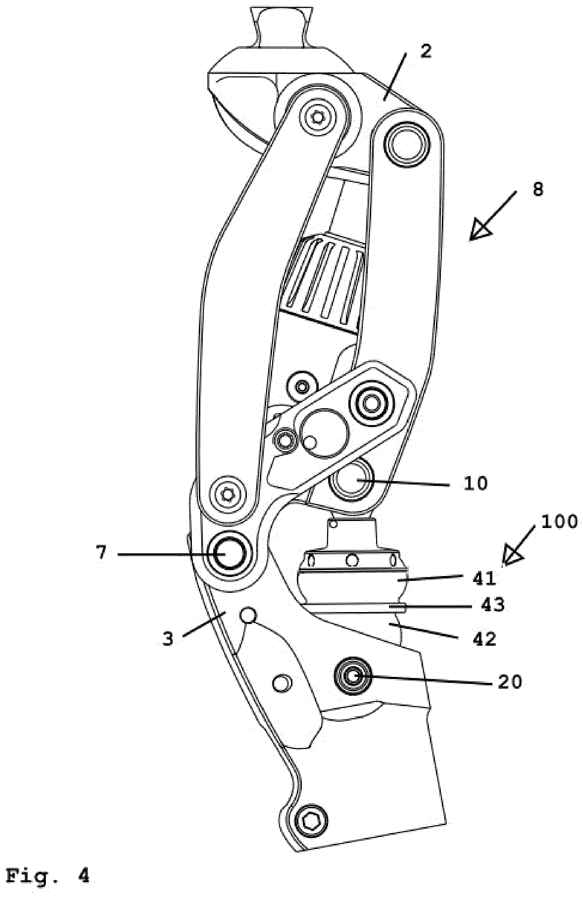

Figur 4 zeigt nur noch das Prothesenkniegelenk ohne Oberschenkelschaft 6 oder Unterschenkelrohr 4 mit dem Oberteil 2, dem Unterteil 3, dem Mehrlenkersystem 8 sowie der Schwenkachse 7. In der Figur 3 ist das Prothesenkniegelenk während einer Standphasenflexion gezeigt, bei der das Prothesenkniegelenk nicht mit dem Mehrlenkersystem 8 einbeugt, sondern um die Schwenkachse 7 in Uhrzeigerrichtung bei einer Axialbelastung verschwenkt. Der Nutzer oder der Patient sinkt somit bei einer Fersenbelastung oder einer Beugung in der Standphase durch das nachgiebige Dämpfersystem 100 ein, beispielsweise bei dem sogenannten Heel Strike, so dass keine ungedämpfte Krafteinleitung durch das gestreckte Prothesenbein in den Stumpf oder in das Hüftgelenk erfolgt. Der proximale und distale tonnenförmige oder glockenförmige Abschnitt 41, 42 wird durch die Standphasenbelastung und das Aufeinanderzubewegen der proximalen und distalen Befestigungseinrichtungen 10, 20 komprimiert und wölben sich nach außen. Der Abstützabschnitt 43 teilt die beiden Abschnitte 41, 42.

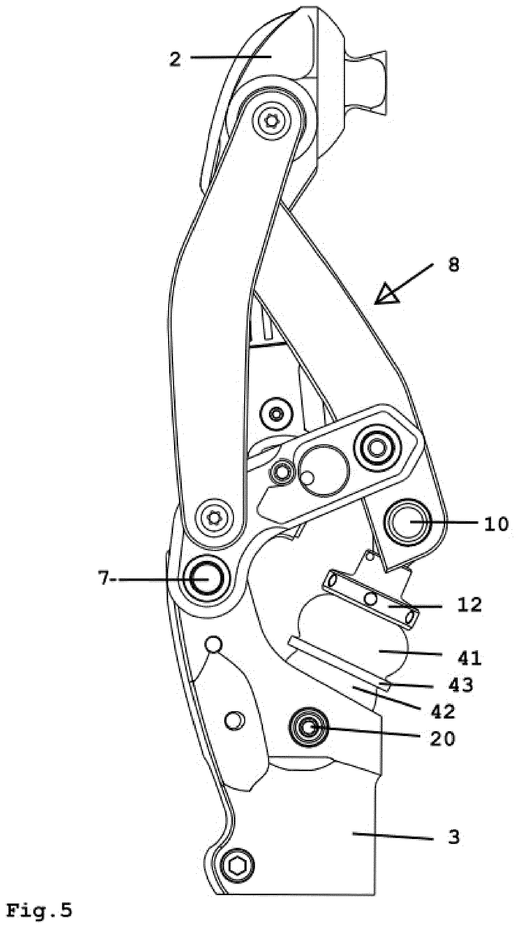

Figur 5 zeigt das Prothesenkniegelenk gemäß Figuren 3 und 4 in einem flektierten Zustand. Das Oberteil 2 ist um 90° gegenüber dem Unterteil 3 verglichen mit der extendierten Stellung gemäß Figur 3 verschwenkt. Es ist zu erkennen, dass das Mehrlenkersystem 8 eine Verlagerung der jeweiligen Lenker zueinander ausgeführt hat. Eine Standphasenbelastung findet nicht mehr statt, das Dämpfersystem 100 ist entspannt und die tonnenförmigen oder glockenförmigen Abschnitte 41, 42 sind in ihre Ausgangsform zurückgekehrt.

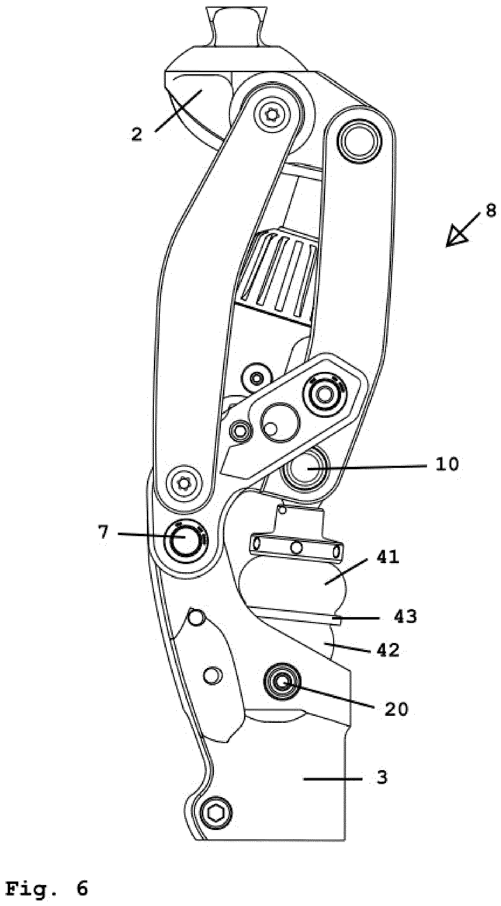

Figur 6 zeigt das Prothesenkniegelenk gemäß Figur 3 in einer vergrößerten Detaildarstellung. Das Prothesenkniegelenk ist vollständig extendiert, es findet keine Standphasenbelastung statt, dementsprechend ist das Dämpfersystem 100 nicht belastet und die beiden Befestigungseirichtungen 10, 20 befinden sich in einem maximal entfernten Zustand.

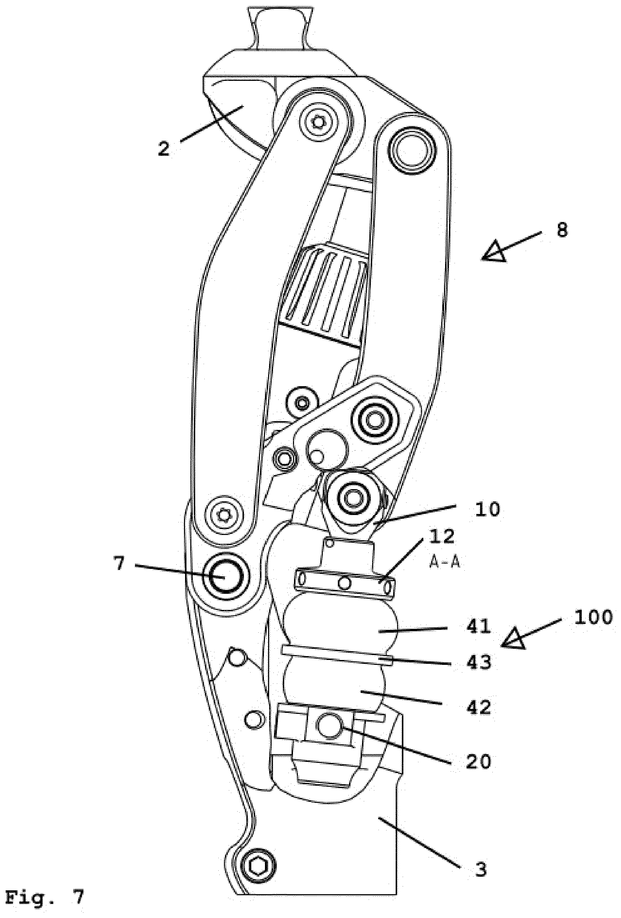

Figur 7 zeigt das Dämpfersystem in der Stellung gemäß Figur 6 im eingebauten Zustand. Teile des Gehäuses des Unterteils 3 sind freigeschnitten, um das Dämpfersystem 100, das im Wesentlichen dem Aufbau gemäß der Figuren 1 und 2 entspricht, gezeigt ist.

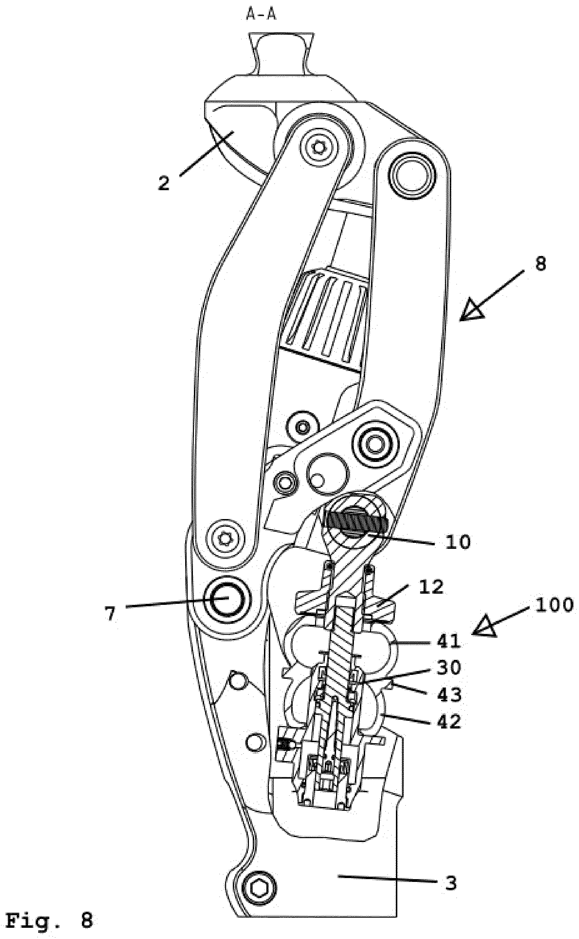

In der Figur 8 ist das Dämpfersystem 100 gemäß Figur 7 in einer Schnittdarstellung gezeigt. Das Dämpfersystem 100 entspricht dabei dem Aufbau gemäß Figur 2.

Alternativ zu einer Umhüllung der ersten Dämpfereinrichtung 30 durch die zweite Dämpfereinrichtung 40 in Gestalt des Tubusstrukturdämpfers besteht die Möglichkeit, dass ein erster Abschnitt 41 oberhalb der ersten Dämpfereinrichtung 30 oder proximal zu der ersten Dämpfereinrichtung 30 und ein zweiter Abschnitt 42 unterhalb oder distal zu der ersten Dämpfereinrichtung 30 angeordnet sein kann, sodass die erste Dämpfereinrichtung 30 in Reihe zwischen zwei Tubusstrukturdämpfer-Abschnitten oder Tubusstrukturdämpfern 40 eingebettet sein kann. Die jeweils proximal und distal angeordneten Tubusstrukturdämpfer 40, Abschnitte oder zweite Dämpfereinrichtungen können mehrere nach außen gewölbte, tonnenförmige und/oder glockenförmige Abschnitte mit unterschiedlichen Durchmessern und/oder unterschiedlichen Höhen aufweisen.

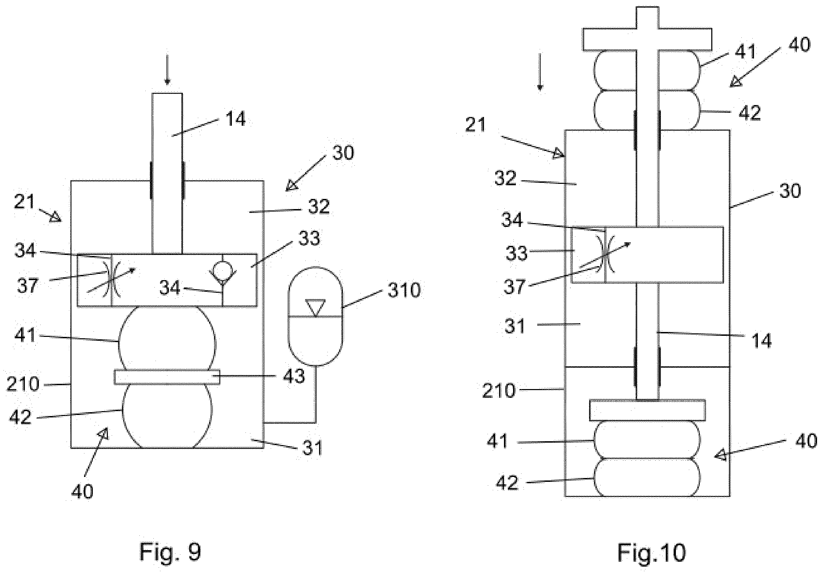

In der Figur 9 ist in einer schematischen Darstellung ein Dämpfersystem mit einer ersten hydraulischen oder pneumatischen Dämpfereinrichtung 30 mit einem Zylinder 21 angedeutet, den eine Zylinderwand in einem Zylindergehäuse 210 umgibt. Innerhalb des Zylindergehäuses 210 ist der Kolben 33 der ersten Dämpfereinrichtung 30 längsverschieblich an einer Kolbenstange 14 angeordnet. Innerhalb sowohl der Flexionskammer 31 als auch in der Extensionskammer 32 ist ein Fluid, beispielsweise ein Hydraulikfluid angeordnet. In dem dargestellten Ausführungsbeispiel ist in der Flexionskammer 31 die zweite Dämpfereinrichtung 40 mit den beiden glockenförmigen Abschnitten 41, 42 des Tubusstrukturdämpfers angeordnet. Die zweite Dämpfereinrichtung 40 liegt an der der Kolbenstange 14 abgewandten Seite des Kolbens 33 an und wird komprimiert, wenn die Kolbenstange 14 zusammen mit dem Kolben 33 beispielsweise bei einer Flexion einer orthopädietechnischen Einrichtung in Richtung auf die Flexionskammer 31 verlagert wird. Dies kann beispielsweise bei der Flexion eines Gelenkes der Fall sein. Um das eindringende Volumen der Kolbenstange 14 in die Extensionskammer 32 zu kompensieren, ist ein Ausgleichsbehälter 310 der Flexionskammer 31 zugeordnet. Wird eine Druckkraft auf die Kolbenstange 14 ausgeübt, wie durch den Pfeil angedeutet, beispielsweise bei einer Flexion eines Orthesen- oder Prothesengelenkes, bewegt sich der Kolben 33 nach unten und komprimiert die erste Dämpfereinrichtung 40. Gleichzeitig wird Hydraulikfluid oder Pneumatikfluid aus der Flexionskammer in das Ausgleichsvolumen 310 gedrückt. Innerhalb des Kolbens 33 sind Überströmleitungen 34 angeordnet, die mit Schaltelementen 37 gekoppelt sind. Ein Schaltelement 37 ist als ein einstellbares Ventil oder eine Drossel ausgebildet, um den Strömungswiderstand durch die Überströmleitung 34 von der Flexionskammer 31 in die Extensionskammer 32 einzustellen. Ebenfalls ist ein Rückschlagventil in einer zweiten Überströmleitung 34 angeordnet, um einen im Wesentlichen ungehinderten Strömungsdurchgang des Hydraulikfluids von der Flexionskammer 31 in die Extensionskammer 32 bereitzustellen. Das Rückschlagventil kann federbelastet sein, sodass es erst bei Erreichen eines Grenzdruckes öffnet und den Durchgang freigibt.

Bei einer Umkehrung der Bewegung, also bei einer Extension des Gelenkes und bei einem Herausfahren der Kolbenstange 14 aus der Extensionskammer 32, strömt das Fluid aus der Extensionskammer 32 durch das Einstellventil 37 durch die Überströmleitung 34 in die Flexionskammer 32. Darüber hinaus findet ein Volumenausgleich durch das Fluid aus dem Ausgleichsbehälter 310 statt.

In dem Ausführungsbeispiel gemäß Figur 9 ist die zweite Dämpfereinrichtung 40 vollständig in dem Fluid innerhalb der ersten Dämpfereinrichtung 30 eingebettet. Der Abstützabschnitt 43 kann an dem Gehäuse 210 oder der Zylinderwand des Hydraulikdämpfers oder Pneumatikdämpfers der ersten Dämpfereinrichtung 30 anliegen. Um das Fluid in den Ausgleichsbehälter 310 strömen zu lassen, kann in dem Abstützabschnitt 43 zumindest ein Durchströmkanal angeordnet sein.

Eine Variante der Erfindung ist in der Figur 10 gezeigt, bei der ebenfalls der Kolben 33 der ersten Dämpfereinrichtung 30 in einem mit Fluid gefüllten Zylinder 21 innerhalb eines Gehäuses 210 längsverschieblich angeordnet ist. Ein Überströmkanal 34 ist in dem Kolben 33 ausgebildet und weist ein Ventil als Schaltelement 33 auf, um den Strömungswiderstand in Extensionsrichtung und/oder Flexionsrichtung zumindest zu reduzieren und gegebenenfalls unterschiedliche Einstellungen für die Extensionsrichtung und/oder Flexionsrichtung zu ermöglichen.

In dem dargestellten Ausführungsbespiel der Figur 10 sind zwei Dämpfereinrichtungen 40 mit der ersten Dämpfereinrichtung 30 kombiniert. An dem oberen Ende des Gehäuses 210 ist eine erste zweite Dämpfereinrichtung 40 angeordnet, die um die Kolbenstange 14 herum angeordnet ist. Bei einer Flexionsbewegung und einem Eindrücken des Kolbens 33 in Richtung auf die Flexionskammer 31 wird die obere zweite Dämpfereinrichtung 40 komprimiert. Ebenso wird eine untere zweite Dämpfereinrichtung 40, die sich in einer von der Flexionskammer 31 abgetrennten Kammer innerhalb des Gehäuses 210 befindet, komprimiert, um die Flexionsbewegung zu dämpfen. Ein Ausgleichsbehälter ist nicht notwendig, da aufgrund der durchgehenden Kolbenstange 14 keine Volumenschwankungen auszugleichen sind. Die untere zweite Dämpfereinrichtung 40 kann sich in einem Hydraulikfluid befinden, ebenfalls ist es möglich, dass die Kammer unterhalb der Flexionskammer 31 ansonsten leer ist oder mit der Umgebungsluft gefüllt ist, die in Austausch mit der Umgebungsluft steht.

Bei einer Extensionsbewegung, also bei einer Aufwärtsbewegung des Kolbens 33 innerhalb des Gehäuses 210, entspannen sich die Elemente der Tubusstrukturdämpfer der zweiten Dämpfereinrichtungen 40.

## Patentansprüche

1. Dämpfersystem für orthopädietechnische Einrichtungen mit einer proximalen Befestigungseinrichtung (10) und einer distalen Befestigungseinrichtung (20), die zueinander verlagerbar miteinander gekoppelt sind, mit einer ersten, zwischen den Befestigungseinrichtungen (10, 20) angeordneten hydraulischen oder pneumatischen Dämpfereinrichtung (30) mit einer Flexionskammer (31) und einer Extensionskammer (32), die voneinander durch einen beweglichen Kolben (33) getrennt und durch zumindest eine Überströmleitung (34) strömungstechnisch miteinander verbunden sind, **dadurch gekennzeichnet, dass** zwischen den Befestigungseinrichtungen (10, 20) eine parallel wirkende, zweite Dämpfereinrichtung (40) mit einem geschwindigkeitsunabhängigen Hystereseverhalten angeordnet ist, die als ein Tubusstrukturdämpfer ausgebildet ist.

2. Dämpfersystem nach Anspruch 1, **dadurch gekennzeichnet, dass** die zweite Dämpfereinrichtung (40) aus einem Elastomer ausgebildet ist.

3. Dämpfersystem nach Anspruch 1 oder 2, **dadurch gekennzeichnet, dass** die zweite Dämpfereinrichtung (40) die erste Dämpfereinrichtung (30) umfänglich umgibt.

4. Dämpfersystem nach einem der voranstehenden Ansprüche, **dadurch gekennzeichnet, dass** die erste Dämpfereinrichtung (30) in ihrem Dämpfungsverhalten einstellbar ausgebildet ist.

5. Dämpfersystem nach einem der voranstehenden Ansprüche, **dadurch gekennzeichnet, dass** der Tubusstrukturdämpfer zumindest einen nach außen gewölbten, tonnenförmigen oder glockenförmigen Abschnitt (41, 42) aufweist.

6. Dämpfersystem nach einem der voranstehenden Ansprüche, **dadurch gekennzeichnet, dass** der Tubussturkturdämpfer mehrere nach außen gewölbte, tonnenförmige und/oder glockenförmige Abschnitte (41, 42) mit unterschiedlichen Durchmessern und/oder unterschiedlichen Höhen aufweist.

7. Dämpfersystem nach einem der voranstehenden Ansprüche, **dadurch gekennzeichnet, dass** der Tubusstrukturdämpfer einen nach innen und/oder außen abstehenden Abstützabschnitt (43) aufweist, der an einem Gehäuse (210) der ersten Dämpfereinrichtung (30) anliegt.

8. Dämpfersystem nach einem der voranstehenden Ansprüche, **dadurch gekennzeichnet, dass** der ersten Dämpfereinrichtung (30) ein Schaltelement (37) zugeordnet ist, das den Strömungswiderstand in der Extensionsrichtung oder Flexionsrichtung zumindest reduziert und in der entgegengesetzten Richtung auf einem eingestellten Strömungswiderstandswert hält.

9. Dämpfersystem nach Anspruch 8, **dadurch gekennzeichnet, dass** in Kompressionsrichtung der zweiten Dämpfereinrichtung (40) der Strömungswiderstand der ersten Dämpfereinrichtung (30) reduziert wird.

10. Dämpfersystem nach einem der voranstehenden Ansprüche, **dadurch gekennzeichnet, dass** das Hystereseverhalten der zweiten Dämpfereinrichtung (40) weicher als das der ersten Dämpfereinrichtung (30) ist.

11. Dämpfersystem nach einem der voranstehenden Ansprüche, **dadurch gekennzeichnet, dass** eine der Befestigungseinrichtungen (10, 20) einen Zylinder (21) zur Aufnahme des Kolbens (33) ausbildet.

12. Dämpfersystem nach einem der voranstehenden Ansprüche, **dadurch gekennzeichnet, dass** die zweite Dämpfereinrichtung (40) innerhalb des Fluids der ersten Dämpfereinrichtung (30) geordnet ist.

13. Dämpfersystem nach Anspruch 6, **dadurch gekennzeichnet, dass** zumindest einer der tonnenförmigen und/oder glockenförmigen Abschnitte (41, 42) oberhalb und zumindest einer der tonnenförmigen und/oder glockenförmigen Abschnitte (41, 42) unterhalb der ersten Dämpfereinrichtung (30) angeordnet ist.

## Claims

1. A damper system for orthopedic devices, having a proximal fastening device (10) and a distal fastening device (20) which are coupled to each other and displaceable relative to each other, having a first hydraulic or pneumatic damper device (30), which is arranged between the fastening devices (10, 20) and has a flexion chamber (31) and an extension chamber (32) which are separated from each other by a movable piston (33) and are fluidically connected to each other by at least one overflow line (34), **characterized in that** a second damper device (40) with parallel action, and with a speed-independent hysteresis behavior, is arranged between the fastening devices (10, 20) and is designed as a tube structure damper.

2. The damper system as claimed in claim 1, **characterized in that** the second damper device (40) is made of an elastomer.

3. The damper system as claimed in claim 1 or 2, **characterized in that** the second damper device (40) surrounds the first damper device (30) circumferentially.

4. The damper system as claimed in one of the preceding claims, **characterized in that** the damping behavior of the first damper device (30) is adjustable.

5. The damper system as claimed in one of the preceding claims, **characterized in that** the tube structure damper has at least one outwardly curved, barrel-shaped or bell-shaped portion (41, 42).

6. The damper system as claimed in one of the preceding claims, **characterized in that** the tube structure damper has a plurality of outwardly curved, barrel-shaped and/or bell-shaped portions (41, 42) with different diameters and/or different heights.

7. The damper system as claimed in one of the preceding claims, **characterized in that** the tube structure damper has an inwardly and/or outwardly projecting support portion (43), which bears on a housing (210) of the first damper device (30).

8. The damper system as claimed in one of the preceding claims, **characterized in that** the first damper device (30) is assigned a switching element (37) which at least reduces the flow resistance in the extension direction or flexion direction and keeps it at a set flow resistance value in the opposite direction.

9. The damper system as claimed in claim 8, **characterized in that** the flow resistance of the first damper device (30) is reduced in the compression direction of the second damper device (40) .

10. The damper system as claimed in one of the preceding claims, **characterized in that** the hysteresis behavior of the second damper device (40) is softer than that of the first damper device (30).

11. The damper system as claimed in one of the preceding claims, **characterized in that** one of the fastening devices (10, 20) forms a cylinder (21) for receiving the piston (33).

12. The damper system as claimed in one of the preceding claims, **characterized in that** the second damper device (40) is arranged within the fluid of the first damper device (30).

13. The damper system as claimed in claim 6, **characterized in that** at least one of the barrel-shaped and/or bell-shaped portions (41, 42) is arranged above the first damper device (30), and at least one of the barrel-shaped and/or bell-shaped portions (41, 42) is arranged below the first damper device (30).

## Revendications

1. Système d'amortissement pour dispositifs orthopédiques comprenant un dispositif de fixation proximal (10) et un dispositif de fixation distal (20), qui sont couplés l'un à l'autre de manière déplaçable l'un par rapport à l'autre, comprenant un premier dispositif d'amortissement hydraulique ou pneumatique (30) disposé entre les dispositifs de fixation (10, 20) et présentant une chambre de flexion (31) et une chambre d'extension (32), qui sont séparées l'une de l'autre par un piston mobile (33) et reliées fluidiquement l'une à l'autre par au moins une conduite de passage (34), **caractérisé en ce qu'**un deuxième dispositif d'amortissement (40) agissant en parallèle et ayant un comportement d'hystérésis indépendant de la vitesse est disposé entre les dispositifs de fixation (10, 20), qui est conçu comme un amortisseur à structure tubulaire.

2. Système d'amortissement selon la revendication 1,
**caractérisé en ce que** le deuxième dispositif d'amortissement (40) est réalisé en élastomère.

3. Système d'amortissement selon la revendication 1 ou 2,
**caractérisé en ce que** le deuxième dispositif d'amortissement (40) entoure périphériquement le premier dispositif d'amortissement (30).

4. Système d'amortissement selon l'une des revendications précédentes, **caractérisé en ce que** le premier dispositif d'amortissement (30) est réglable quant à son comportement d'amortissement.

5. Système d'amortissement selon l'une des revendications précédentes, **caractérisé en ce que** l'amortisseur à structure tubulaire comporte au moins une portion (41) en forme de tonneau ou de cloche, bombée vers l'extérieur.

6. Système d'amortissement selon l'une des revendications précédentes, **caractérisé en ce que** l'amortisseur à structure tubulaire présente plusieurs portions (41, 42) en forme de tonneau et/ou de cloche, bombées vers l'extérieur, de diamètres différents et/ou de hauteurs différentes.

7. Système d'amortissement selon l'une des revendications précédentes, **caractérisé en ce que** l'amortisseur à structure tubulaire présente une portion d'appui (43) en saillie vers l'intérieur et/ou vers l'extérieur, qui s'appuie contre un boîtier (210) du premier dispositif d'amortissement (30).

8. Système d'amortissement selon l'une des revendications précédentes, **caractérisé en ce qu'**un élément de commutation (37) est associé au premier dispositif d'amortissement (30), lequel réduit au moins la résistance à l'écoulement dans la direction d'extension ou dans la direction de flexion et la maintient à une valeur de résistance à l'écoulement réglée dans la direction opposée.

9. Système d'amortissement selon la revendication 8,
**caractérisé en ce que** la résistance à l'écoulement du premier dispositif d'amortissement (30) est réduite dans la direction de compression du deuxième dispositif d'amortissement (40).

10. Système d'amortissement selon l'une des revendications précédentes, **caractérisé en ce que** le comportement d'hystérésis du deuxième dispositif d'amortissement (40) est plus doux que celui du premier dispositif d'amortissement (30).

11. Système d'amortissement selon l'une des revendications précédentes, **caractérisé en ce que** l'un des dispositifs de fixation (10, 20) constitue un cylindre (21) destiné à recevoir le piston (33).

12. Système d'amortissement selon l'une des revendications précédentes, **caractérisé en ce que** le deuxième dispositif d'amortissement (40) est disposé à l'intérieur du fluide du premier dispositif d'amortissement (30).

13. Système d'amortissement selon la revendication 6,
**caractérisé en ce qu'**au moins l'une des portions (41, 42) en forme de tonneau et/ou de cloche est disposée au-dessus du premier dispositif d'amortissement (30) et au moins l'une des portions (41, 42) en forme de tonneau et/ou de cloche est disposée au-dessous.
